# EUROPEAN PATENT APPLICATION

(11) **EP 3 808 394 A1**
(43) Date of publication of application: **21.04.2021**
(21) Application number: 19203249.8
(22) Date of filing: 15.10.2019
(51) Int. Cl.: A61M 5/28, A61M 5/315, A61M 5/32, A61B 5/15, A61M 5/31

(54) **SYRINGE DEVICE**

(71) Applicant: Lin, Chung-Sing, Tainan 710 (TW)
(72) Inventor: Lin, Chung-Sing, Tainan 710 (TW)
(74) Representative: Brown, Michael Stanley

(57) **Abstract**

A syringe device 1 includes a barrel 10 and a compressible portion 11 is connected to one end of the barrel 1. A positioning board 12 is located between the barrel 1 and the compressible portion 11. The positioning board 12 has a hole 120 defined therethrough. A seat 121 is formed to the underside of the positioning board 12 and has a recess 122 and multiple guide grooves 123 which communicate with the recess 122. A hold part 13 is connected to the distal end of the compressible portion 22 and has a penetrating face 130. Two flanges 131 respectively extend diametrically from the hold part 13. A needle 14 is connected to the positioning board 12 and communicates with the recess 122. The tip 140 of the needle 14 is located within the compressible portion 11 and beneath the penetrating face 130. By axially compressing the compressible portion 11 and the barrel 1, the needle 14 penetrates through the penetrating face 130 and extends beyond the hole 120 to eject medicine to the patient.

## Description

### BACKGROUND OF THE INVENTION

### 1. Fields of the invention

The present invention relates to a syringe device, and more particularly, to a syringe device with a hidden needle in a compressible portion on the barrel.

### 2. Descriptions of Related Art

The conventional syringe device generally includes a barrel, a needle and a plunger. The plunger is movably inserted into the barrel so as to inject medicine in the barrel into the patient body via the needle. The needle is a thin and elongate tube with a tip on one end thereof. When the injection is completed, the nurses will insert the needle of the syringe into the protective cover. It is a risk for the nurses if the needle accidentally touches the nurses' skin during the insertion of the needle back into the protective cover. Besides, the amount of the protective cover is significant and that create a lot of medical garbage.

The present invention intends to provide a syringe device wherein the needle can be retrieved into the compressible portion after use.

### SUMMARY OF THE INVENTION

The present invention relates to a syringe device and comprises a barrel, and a compressible portion is connected to the first end of the barrel. A positioning board is located between the barrel and the compressible portion, and has a hole defined therethrough. A seat is formed to the underside of the positioning board and has a recess and multiple guide grooves which communicate with the recess. A projection extends radially outward from outside of the positioning board. A hold part is connected to the distal end of the compressible portion and includes a penetrating face. Two flanges respectively extend diametrically from the hold part, and each flange having a hook so as to be detachably hooked to the projection of the positioning board when the barrel and the compressible portion are compressed. A needle is connected to the positioning board and communicates with the recess. The tip of the needle is located within the compressible portion and beneath the penetrating face.

Preferably, a protrusion protrudes from the inner bottom of the second end of the barrel. The protrusion is located corresponding to the recess of the seat.

Preferably, the barrel is a bellow tube.

Preferably, the compressible portion is cone shaped.

Preferably, the barrel receives medicine therein.

The primary object of the present invention is to provide a syringe device that combines the needle, the barrel and the medicine as a one part, and the nurses do not need to discard the needle or using a protective cover. The needle is accommodated in the compressible portion on the barrel after being used.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is a perspective view to show the syringe device of the present invention;
Fig. 2 is a cross sectional view of the syringe device of the present invention;
Fig. 3 shows that a user's hand holds the syringe device of the present invention;
Fig. 4 shows that the needle extends beyond the hold part when the compressible portion is compressed;
Fig. 5 is a cross sectional view of the syringe device as disclosed in Fig. 4;
Fig. 6 is a perspective view to show that the compressible portion and the barrel are both compressed, and
Fig. 7 illustrates that medicine in the barrel is ejected from the needle

### DETAILED DESCRIPTION OF THE PREFERRED EMBODIMENT

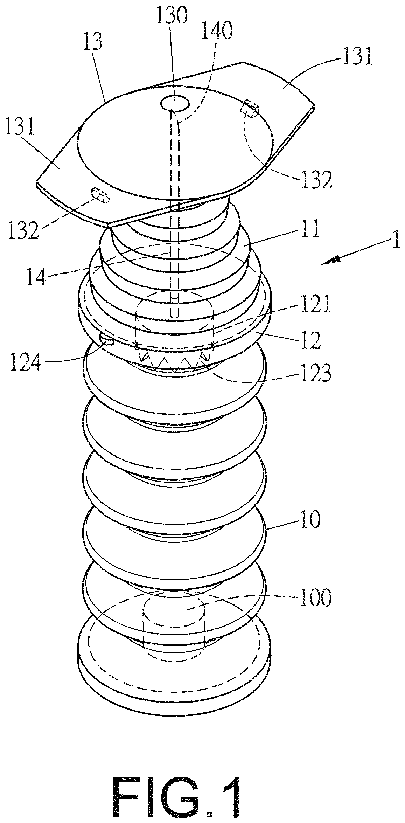

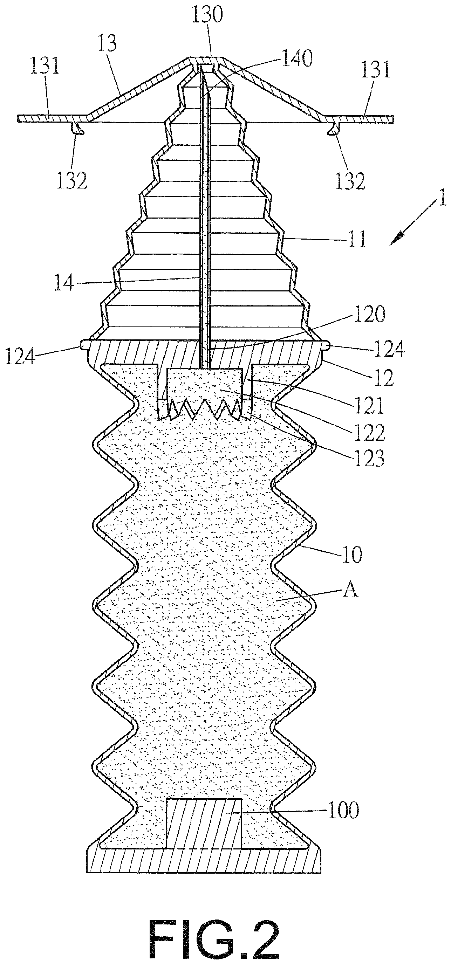

Referring to Figs. 1 and 2, the syringe device 1 of the present invention comprises a barrel 10 which is a bellow tube, and a compressible portion 11 is connected to the first end of the barrel 10. The compressible portion 11 is a cone-shaped bellow. A protrusion 100 protrudes from the inner bottom of the second end of the barrel 10. A positioning board 12 is located between the barrel 10 and the compressible portion 11. The positioning board 12 includes a hole 120 defined therethrough. A seat 121 is formed to the underside of the positioning board 12 and has a recess 122 and multiple guide grooves 123 which communicate with the recess 122. The hole of the positioning board communicates with the recess 122. The protrusion 100 is located corresponding to the recess 122 of the seat 121. Two projections 124 extend diametrically and radially outward from outside of the positioning board 12.

A hold part 13 is connected to the distal end of the compressible portion 11 and includes a penetrating face 130. Two flanges 131 respectively extend diametrically from the hold part 13. Each flange 131 has a hook 132 which is detachably hooked to the projections 124 of the positioning board 12. A needle 14 is inserted through the hole 120 of the positioning board 12 and communicates with the recess 122. The tip 140 of the needle 14 is located within the compressible portion 13 and beneath the penetrating face 130. The barrel 10 receives medicine "A" therein.

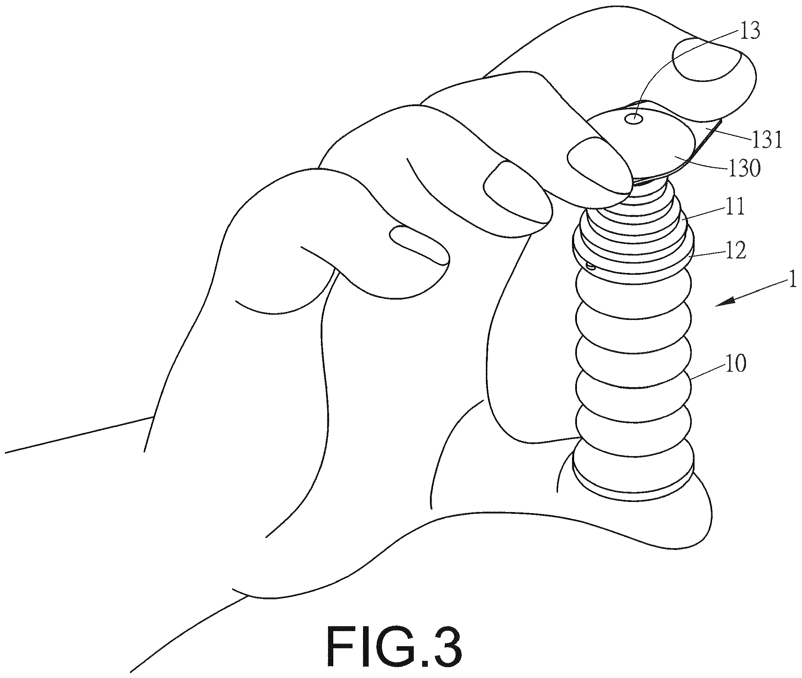

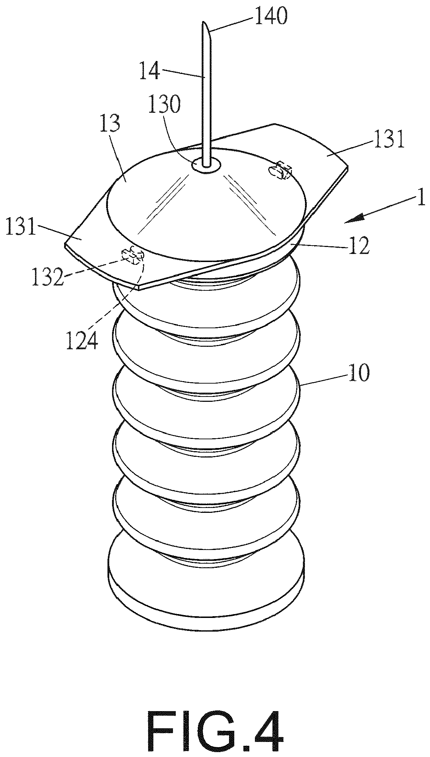

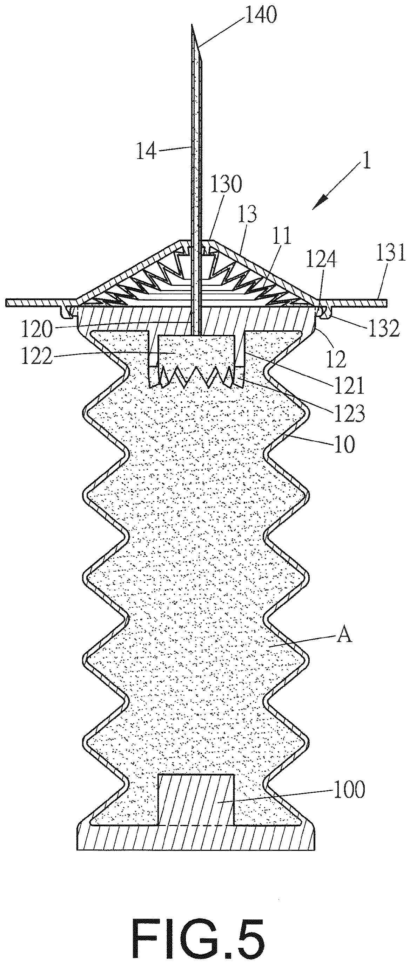

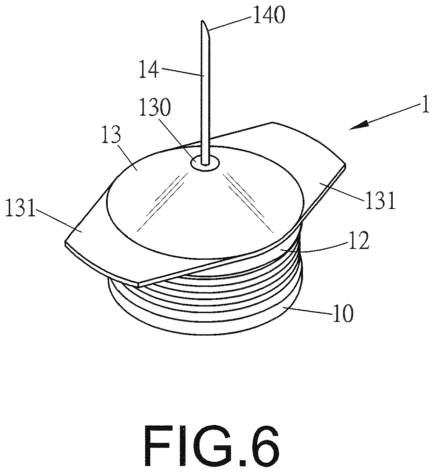

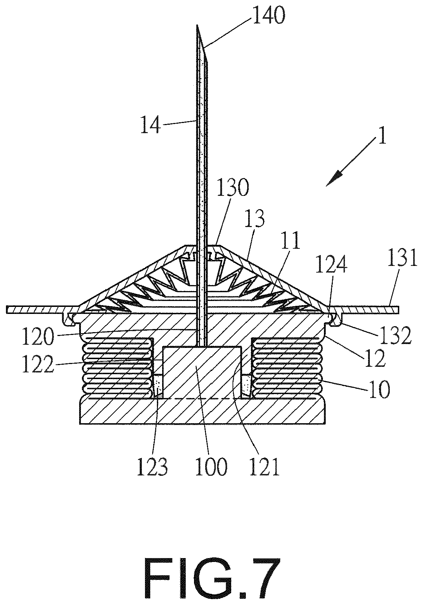

As shown in Figs. 3 to 7, when in use, the nurse contacts the second end of the barrel 10 by the thumb, and two fingers put on the two flanges 131 of the hold part 13 as shown in Fig. 3. By pushing the flanges 131 and the bottom of the barrel 10 axially toward each other, the hold part 13 compresses the compressible portion 11 which is collapsible toward the positioning board 12. The tip 140 of the needle 14 penetrates through the penetrating face 130 of the hold part 13 and extends beyond the hold part 13 as shown in Fig. 4. The projections 124 are hooked by the hooks 132 as show in Fig. 5 so that the hold part 13 does not jump upward and separate from the positioning board 12. The needle 14 can be inserted into a patient body (not shown). The barrel 10 is then compressed to eject the medicine "A" in the barrel 10 to the patient (not shown) via the recess 122 and the guide grooves 122 and the needle 14 as shown in Figs. 6 and 7. The protrusion 100 is engaged with the recess 122 of the seat 121 to completely push the medicine "A" out from the barrel 10. When the ejection of medicine "A" is completed, the barrel 10 and the hold part 13 are released, the barrel 10 returns back to its initial or expanded status as shown in Fig. 4. Then the engagement of the hooks 132 and the projections 124 are released, the compressible portion 11 returns to its initial or expanded status. The needle 14 is hidden by the hold part 13 and accommodated in the compressible portion 11.

The nurses do not need to deal with the needle 14 after use to protect the nurses conveniently.

## Claims

1. A syringe device 1 **characterized in that** it includes:
a barrel 10, a compressible portion 11 connected to a first end of the barrel 1, a positioning board 12 located between the barrel 1 and the compressible portion 11, the positioning board 12 having a hole 120 defined therethrough, a seat 121 formed to an underside of the positioning board 12 and having a recess 122 and multiple guide grooves 123 which communicate with the recess 122, a projection 124 extending radially outward from outside of the positioning board 12;
a hold part 13 connected to a distal end of the compressible portion 11 and having a penetrating face 130, two flanges 131 respectively extending diametrically from the hold part 13, each flange 131 having a hook which is detachably hooked to the projection 124 of the positioning board 12, and
a needle 14 connected to the positioning board 12 and communicating with the recess 122, a tip 140 of the needle 14 located within the compressible portion 22 and beneath the penetrating face 130.

2. The syringe device 1 as claimed in claim 1, wherein a protrusion 100 protrudes from an inner bottom of a second end of the barrel 10, the protrusion 100 is located corresponding to the recess 122 of the seat 121.

3. The syringe device 1 as claimed in claim 1, wherein the barrel 10 is a bellow tube.

4. The syringe device 1 as claimed in claim 1, wherein the compressible portion 11 is cone shaped.

5. The syringe device 1 as claimed in claim 1, wherein the barrel 10 receives medicine therein.
